(19) Europäisches Patentamt
European Patent Office
Office européen des brevets

(11) Veröffentlichungsnummer: **0 129 814**
**B1**

(12) # EUROPÄISCHE PATENTSCHRIFT

(45) Veröffentlichungstag der Patentschrift: **10.12.86**

(51) Int. Cl.⁴: **C 07 D 301/19**, C 07 D 303/04 // C07C178/00, C07C179/087, B01J21/06

(21) Anmeldenummer: **84106932.1**

(22) Anmeldetag: **16.06.84**

(54) **Verfahren zur Herstellung von Oxiranen.**

(30) Priorität: **24.06.83 DE 3322745**

(43) Veröffentlichungstag der Anmeldung: **02.01.85 Patentblatt 85/1**

(45) Bekanntmachung des Hinweises auf die Patenterteilung: **10.12.86 Patentblatt 86/50**

(84) Benannte Vertragsstaaten: **BE DE FR GB IT NL**

(56) Entgegenhaltungen:
**DE - A - 2 015 503**
**US - A - 3 351 635**

(73) Patentinhaber: **BASF Aktiengesellschaft, Carl-Bosch-Strasse 38, D-6700 Ludwigshafen (DE)**

(72) Erfinder: **Fischer, Rolf, Dr., Bergstrasse 98, D-6900 Heidelberg (DE)**
Erfinder: **Weitz, Hans-Martin, Dr., Auf dem Koeppel 40, D-6702 Bad Duerkheim (DE)**
Erfinder: **Rieber, Norbert, Dr., Liebfrauenstrasse 1 c, D-6800 Mannheim 51 (DE)**
Erfinder: **Boehm, Heinrich, Dr., Keplerweg 2, D-6708 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 129 814 B1

ACTORUM AG

**Beschreibung**

Die vorliegende Erfindung betrifft ein neues Verfahren zur Herstellung von Oxiranen durch Umsetzung von olefinisch ungesättigten Verbindungen mit organischen Peroxiden in flüssiger Phase sowie in Gegenwart von Epoxidierungskatalysatoren.

Aus zahlreichen Veröffentlichungen ist es allgemein bekannt, olefinisch ungesättigte Verbindungen mit organischen Peroxiden und Epoxidierungskatalysatoren in flüssiger Phase gemäss dem allgemeinen Reaktionsschema

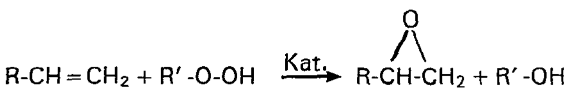


R, R′ = organische Reste

in die entsprechenden Oxirane und Hydroxyl-Verbindungen zu überführen.

Hierbei ist grundsätzlich zwischen zwei verschiedenen verfahrenstechnischen Arbeitsrichtungen zu unterscheiden, nämlich derjenigen der homogenen und derjenigen der heterogenen Katalyse.

Bei der homogenen Katalyse, die vor allem in der US-PS 3 351 635 ausführlich beschrieben ist, finden solche Epoxidierungskatalysatoren Verwendung, die im Reaktionsgemisch löslich sind, und zwar insbesondere Verbindungen des Molybdäns und Wolframs, wie beispielsweise die Naphthenate. Mit Verbindungen anderer, an sich prinzipiell geeigneter Katalysatormetalle, darunter des Titans, erzielt man hingegen deutlich schlechtere Olefin-Umsätze und Oxiran-Ausbeuten. Als Peroxide dienen hierbei vornehmlich tert.-Butylhydroperoxid, Cumolhydroperoxid und das 1-Phenylethan-1-hydroperoxid. Andere Peroxide wie das Cyclohexanonperoxid (Cyclohexanolhydroperoxid)

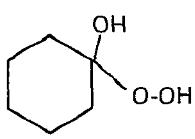

das Sauerstoff-Oxidationsprodukt des Cyclohexanols, liefert im Vergleich dazu hingegen nur unbefriedigende Ergebnisse (s. Beispiel 5 der US-PS cit.).

Insgesamt folgt aus den Untersuchungen über die homogene Katalyse der in Rede stehenden Reaktion, dass sich hierfür weder Titanverbindungen als Epoxidierungskatalysatoren noch Cyclohexanonperoxid als Sauerstoffüberträger empfehlen. Abgesehen davon bietet die homogene Katalyse verfahrenstechnische Schwierigkeiten, so dass der heterogenen Katalyse für technische Zwecke ohnehin der Vorzug zu geben ist.

Für die heterogene Katalyse sind vor allem Trägerkatalysatoren mit Titandioxid als aktiver Masse und Siliciumdioxid oder Silikaten als Trägermaterial bekannt geworden (DE-AS 2 015 503), und als Peroxide verwendet man bei dieser Verfahrensweise Peralkohole wie tert.-Butylhydroperoxid und vor allem 1-Phenylethan-1-hydroperoxid (Ethylbenzolhydroperoxid).

Obwohl man hierbei hohe Peroxidumsätze und auch relativ hohe Selektivitäten bezüglich der Oxirane erzielen kann, lässt das Verfahren in wirtschaftlicher Hinsicht zu wünschen übrig. Die genannten Peralkohole, die durch Sauerstoff-Oxidation aus den entsprechenden Kohlenwasserstoffen (z.B. Isobutan und Ethylbenzol) zugänglich sind, gehen nämlich in die Alkohole über, welche nicht wieder einfach mit Sauerstoff oder Luft in die Perverbindungen zurückverwandelt werden können. Für einen Kreisprozess müsste man daher die Alkohole dehydratisieren und die hierbei entstehenden Olefine (z.B. Isobuten und Styrol) wieder zu den ursprünglichen Kohlenwasserstoffen hydrieren.

Bedenkt man, dass man beispielsweise Styrol grosstechnisch durch Dehydrieren von Ethylbenzol gewinnt, so leuchtet es ohne weiteres ein, dass es wirtschaftlich widersinnig wäre, Ethylbenzol aus Styrol herzustellen, wie es ein Epoxidationsverfahren mit geschlossenem Kreislauf erfordern würde.

Aus diesem Grunde verzichtet man auf einen Kreislauf und gewinnt das Olefin als Kopplungsprodukt. Das Kopplungsprodukt, hauptsächlich Styrol, kann aber nur zu dem Preis bewertet werden, wie er sich nach den billigen grosstechnischen Verfahren ergibt. Da dieser Preis erheblich geringer ist als der Preis über das Epoxidationsverfahren, verteuert sich das Oxiran entsprechend.

Die vorliegende Erfindung entspringt daher dem allgemeinen Bestreben, die Wirtschaftlichkeit des Verfahrens zur Herstellung von Oxiranen aus olefinisch ungesättigten Verbindungen und Peroxiden zu erhöhen, sei es durch einen kostengünstigeren Kreisprozess und/oder durch ein wertvolleres Kopplungsprodukt.

Demgemäss wurde gefunden, dass man Oxirane durch Umsetzung der entsprechenden olefinisch ungesättigten Verbindungen mit organischen Peroxiden in flüssiger Phase sowie in Gegenwart von Trägerkatalysatoren als Epoxidierungskatalysatoren, die in der aktiven Masse vornehmlich Titandioxid enthalten und deren Trägermaterial vornehmlich aus Siliciumdioxid oder Silikaten besteht, wirtschaftlich herstellen kann, wenn man hierzu Cyclohexanonperoxid als organisches Peroxid verwendet.

Das gute Gelingen dieses Verfahrens, welches, bezogen auf eingesetztes Peroxid, Oxiran-Ausbeuten zwischen 60 und 80% liefert, ist bemerkenswert, weil man nach Beispiel 5 der US-PS 3 351 635 bei der homogen-katalytischen Umsetzung mit Cyclohexanonperoxid nur eine Oxiran-Ausbeute von 38% erzielt, und weil Hydroxyhydroperoxide, zu denen das Cyclohexanonperoxid zählt, in der grundlegenden Untersuchung von Pralus et al. in «Fundamental Research in Homogeneous Catalysis», Band 3, Plenum Press New York/London 1979, Seite 336, als Epoxidierungsagentien generell als unwirksam bezeichnet werden.

Ein weiterer bemerkenswerter Aspekt ist die Tatsache, dass das Cyclohexanonperoxid praktisch quantitativ in das Wertprodukt Cyclohexanon übergeht.

Der sich hieraus ergebende Fortschrift des erfindungsgemässen Verfahrens wird besonders deutlich, wenn man bei der Wirtschaftlichkeitsbetrachtung von den Einsatzstoffen Cyclohexan und Olefin ausgeht. In diesem Falle resultiert aus den einzelnen, im übrigen verfahrenstechnisch unproblematischen Verfahrensschritten

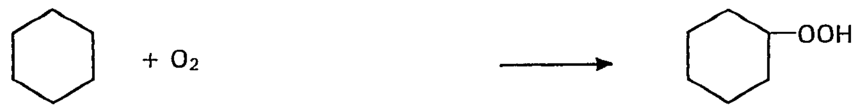
(1)

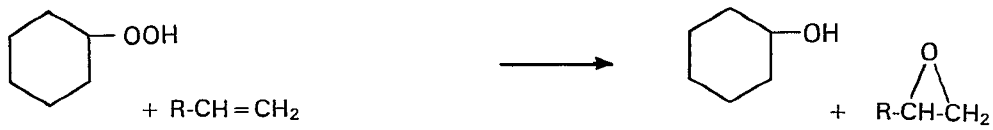
(2)

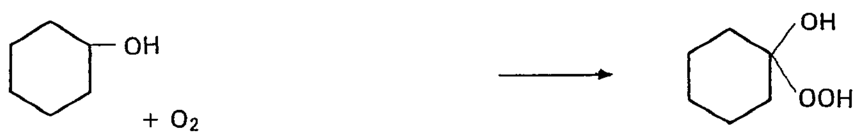
(3)

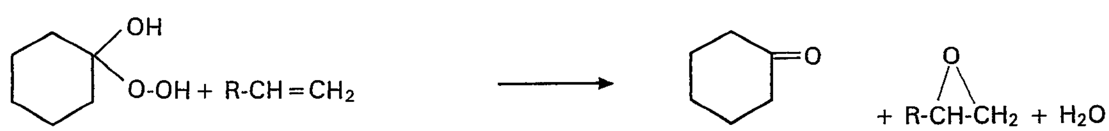
(4)

die Bruttoreakttion

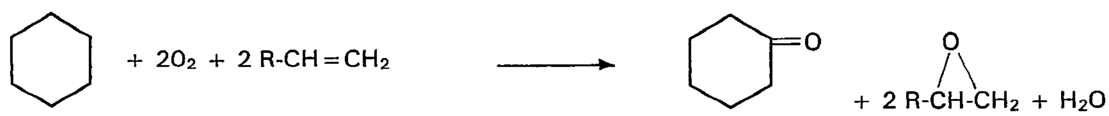
(5)

d.h. man erhält pro Mol Kohlenwasserstoff (Cyclohexan) theoretisch 2 mol Oxiran und nicht nur 1 mol wie im Faller des von Ethylbenzol ausgehenden Coproduktionsverfahrens. Darüber hinaus ist das hier entstehende Kopplungsprodukt, das Cyclohexanon, nicht nur an sich besonders wertvoll, sondern auch in den Herstellkosten praktisch nicht teurer als bei direkter Herstellung aus Cyclohexan. Aus diesem Grunde wird es sich in der Regel nicht empfehlen, das Cyclohexanon zu hydrieren und das dabei erhältliche Cyclohexanol wieder in das Verfahren zurückzuführen.

Die hohe Wirtschaftlichkeit beruht des weiteren auch darauf, dass das Cyclohexanon in Stufe (4) von selbst entsteht und keine zusätzlichen Verfahrensschritte erfordert, wie beispielsweise die Dehydratisierung des Phenylethanols zum Styrol im Falle des von Ethylbenzol ausgehenden Coproduktionsverfahrens.

Das Cyclohexanonperoxid ist in bekannter Weise erhältlich, und zwar technisch am einfachsten, indem man Sauerstoff oder Luft bei 50-160°C bis zu einer Peroxid-Konzentration von etwa 0,1-25 Gew.-% durch Cyclohexanol leitet. Legt man hierbei von vornherein etwas Cyclohexanonperoxid vor, wird die Peroxidbildung beschleunigt.

Diese das Cyclohexanonperoxid enthaltenden Cyclohexanol-Lösungen werden sodann in an sich bekannter Verfahrensweise bei 20-150°C mit der olefinisch ungesättigten Verbindung zu Cyclohexanon und dem Oxiran umgesetzt, wobei es zweckmässig ist, die olefinisch ungesättigte Verbindung in einem Überschuss von 0,1-10 mol über das Peroxid einzusetzen.

Das Arbeiten unter erhöhtem Druck ist im allgemeinen nicht erforderlich, kann sich aber empfehlen, wenn die eingesetzte olefinisch ungesättigte Verbindung bei der Reaktionstemperatur unter Normaldruck gasförmig ist.

Falls die Reaktionspartner, abgesehen vom Katalysator, keine homogen flüssige Phase bilden, verwendet man zweckmässigerweise ein inertes Lösungsmittel mit, z.B. Toluol, Dioxan, Cyclohexan oder Ethylacetat.

Die Aufarbeitung der Reaktionsgemische auf ihre Komponenten Cyclohexanol, Cyclohexanon, olefinisch ungesättigte Verbindung, das Oxiran, Wasser und gegebenenfalls ein Lösungsmittel sowie geringe

Mengen von Nebenprodukten kann nach Abtrennung des Katalysators wie üblich erfolgen, und zwar in der Regel durch fraktionierte Destillation.

Ist das entstehende Oxiran sehr reaktiv, kann es sich empfehlen, das Reaktionswasser während der Umsetzung laufend destillativ aus dem Reaktionsgemisch zu entfernen, etwa mit Cyclohexan als Schleppmittel, damit man nicht anstelle des Oxirans das entsprechende vicinale Glycol in grösseren Anteilen erhält.

Die Bereitung der Cyclohexanonperoxid-Lösung aus Cyclohexanol und Sauerstoff oder Luft und die Epoxidierung der olefinisch ungesättigten Verbindung können, sofern letztere unter den Reaktionsbedingungen nicht selber unmittelbar mit Sauerstoff reagiert, auch gemeinsam vorgenommen werden. Das gleiche gilt für die besonders zweckmässige Ausführungsform des Verfahrens, bei der man zur Herstellung der Cyclohexanonperoxidlösung nicht vom Cyclohexanol sondern vom Cyclohexan ausgeht, wobei man gemäss der obigen Bruttogleichung (5) pro Mol Cyclohexan zwei Mol Oxiran erzeugen kann.

Da es sich bei dem erfindungsgemässen Verfahren um eine heterogene Katalyse handelt, lässt es sich nach den hierfür bekannten Techniken besonders einfach kontinuierlich gestalten.

Das Verfahren ist prinzipiell von der Art der eingesetzten olefinisch ungesättigten Verbindung unabhängig und eignet sich daher grundsätzlich zur Herstellung beliebiger Oxirane. Bedingt oder nicht geeignet sind, wie sich allerdings von selbst versteht, lediglich solche olefinisch ungesättigten Verbindungen, die Substituenten tragen, welche in grösserem Umfang zu Nebenreaktionen Anlass geben, also etwa saure oder basische Gruppen.

Die olefinisch ungesättigten Verbindungen können des weiteren auch zwei oder mehrere -C=C-Gruppierungen enthalten; in diesem Falle entstehen die entsprechenden Bis- und Polyoxirane.

Unter den olefinisch ungesättigten Verbindungen sind in erster Linie die $C_2$-$C_{20}$-Olefine zu nennen. Hierbei können die $C_4$-$C_{20}$-Olefine unverzweigt und verzweigt sein, und die Doppelbindung kann innenständig oder endständig sein. Besondere Bedeutung hat das Propylen als Vorstufe für das im grosstechnischen Massstab benötigte Propylenoxid.

Weiterhin kommen in Betracht:

— Cycloolefine wie Cyclopenten, Cyclohexen, Cycloocten und Cyclododecen

— Bisolefine wie Buta-1,3-dien und Hexa-1,5-dien

— araliphatische Olefine wie Styrol und Stilben

— Vinylalkylether wie Vinylethylether und Vinylester wie Vinylacetat

— Allylalkylether wie Allylmethylether und Allylester wie Allylacetat

— Acrylsäure- und Methacrylsäureester, z.B. die Methylester.

Allgemein können die olefinisch ungesättigten Verbindungen Substituenten wie Halogen, die Nitrogruppe, die Cyangruppe, Alkoxygruppen und Carbalkoxygruppen tragen. Im Falle von Hydroxyl- und Oxogruppen in Aldehyd- oder Ketonfunktion wäre durch Vorversuche festzustellen, ob und inwieweit diese unter den Reaktionsbedingungen zu Nebenreaktionen Anlass geben.

Die für das erfindungsgemässe Verfahren zu verwendenden Trägerkatalysatoren sind bekannt und in bekannter Weise erhältlich (s. z.B. DE-AS 20 15 503). Vorzugsweise sollen sie aus 0,2-50 Gew.% $TiO_2$ als aktiver Masse und aus 50-98,8 Gew.-% als Trägermaterial bestehen.

Die aktive Masse kann ferner noch geringe Mengen an Promotoren wie Erdalkaliverbindungen, insbesondere Oxide des Magnesiums, Calciums, Strontiums oder Bariums enthalten, und das Trägermaterial kann gänzlich oder zum Teil durch Silikate wie Magnesium- oder Alumosilikate ersetzt sein. Bevorzugtes Trägermaterial ist $SiO_2$ mit einer inneren Oberfläche von 100-500 $m^2/g$. Ferner soll das Trägermaterial nach Möglichkeit keine sauren oder basischen Zentren aufweisen, also neutral reagieren.

Zur Herstellung der Trägerkatalysatoren kann man wie üblich verfahren, indem man die Teilchen, deren grösste Abmessung in der Regel etwa 0,1 bis 2 mm beträgt, mit einer wässrigen oder methanolischen Lösung eines Titansalzes, z.B. von basischem Titanacetat, sowie gegebenenfalls von Verbindungen der Promotormetalle in den gewünschten Mengenverhältnissen imprägniert, trocknet und danach zur Überführung des Titansalzes in das Titandioxid im Luftstrom auf 350-800°C erhitzt. Lässt sich nicht alles Titansalz auf einmal auf den Träger aufbringen, ist der Imprägnier- und Trockenvorgang gegebenenfalls entsprechend oft zu wiederholen.

Besonders wirksam hinsichtlich der Oxiran-Selektivität sind solche Katalysatoren, die nach Bereitung des $TiO_2/SiO_2$-Grundkatalysators noch mit organischen Silicium-Verbindungen wie Hexamethyldisilazan nachbehandelt wurden, wie es in der DE-OS 23 11 822 beschrieben ist. Zur weiteren Aktivierung dieser nachbehandelten Katalysatoren ist ferner ein kurzzeitiges Erhitzen im Luftstrom auf 200-500°C vorteilhaft.

Die für das erfindungsgemässe Verfahren wirtschaftlich optimale Menge der Epoxidierungskatalysatoren richtet sich nach dem Gehalt an aktiver Masse, deren Teilchengrösse und nach der Art der Epoxidierungsreaktion. Sie kann daher nicht allgemeingültig angegeben werden, liegt aber in der Regel zwischen 1-200 g pro Liter Reaktionsvolumen und ist im Bereich dieser Richtwerte unschwer durch einige Vorversuche zu ermitteln.

*Beispiel A*

Herstellung eines $TiO_2/SiO_2$-Trägerkatalysators

500 g Silikagel der Teilchengrösse von 0,5-1 mm und der inneren Oberfläche von 456 $m^2/g$ wurden mit einer Lösung aus 36,5 g basischem Titanacetat ($Ti_2OAc_6$) und 500 ml Methanol imprägniert und am Rotationsverdampfer bei 90°C und 27 mbar abs. zur Trockene eingedampft, wonach die Masse 2 Stunden lang im Luftstrom auf 800°C erhitzt wurde.

Die Menge des so erhaltenen Katalysators betrug 369 g, er enthielt 1,8 Gew.-% $TiO_2$.

*Beispiel B*

Herstellung eines silylierten $TiO_2/SiO_2$-Trägerkatalysators

185 g des Katalysators gemäss Beispiel A wurden

eine Stunde lang in einer siedenden Lösung aus 25 g Hexamethyldisilazan und 1250 ml n-Heptan erhitzt, wonach das Heptan bei 80°C und 27 mbar abdestilliert wurde.

Anschliessend wurde der Katalysator zur weiteren Aktivierung noch 50 min lang auf 320 bis 360°C erhitzt.

*Beispiel C*

Herstellung einer cyclohexanolischen Cyclohexanonperoxid-Lösung

In 600 g Cyclohexanol, die als Starter 3 g Cyclohexanonperoxid enthielten, wurden im Laufe von 2,5 h bei 115°C 12,5 l Sauerstoff eingeleitet. Danach hatte die Lösung, wie durch iodometrische Titration festgestellt wurde, einen Gehalt von 5,2 Gew.-% Cyclohexanonperoxid entsprechend einer molaren Konzentration von 0,37 mol/l oder 0,39 mol/kg.

*Beispiel 1*

Herstellung von Propylenoxid (Methyloxiran)

Ein Gemisch aus 10 g (238 mmol) Propylen, 61 g (= 24 mmol Peroxid) der Cyclohexanonperoxid-Lösung gemäss Beispiel C und 5 g des Katalysators gemäss Beispiel A wurde 30 min lang im Schüttelautoklaven auf 80°C erhitzt.

Das Reaktionsgemisch enthielt kein Peroxid mehr und die Ausbeuten an Propylenoxid und Cyclohexanon betrugen nach der GC-Analyse 60 bzw. 97 %, jeweils bezogen auf das eingesetzte Peroxid.

*Beispiel 2*

Herstellung von 1,2-Octenoxid (Hexyloxiran)

Ein Gemisch aus 15 g (134 mmol) Oct-1-en, 20 g Cyclohexanol und 5 g des Katalysators gemäss Beispiel B wurde bei 80°C unter Rühren innerhalb von 30 min mit 34 g (= 13,4 mmol Peroxid) der Cyclohexanonperoxid-Lösung gemäss Beispiel C versetzt. Nach weiteren 5 min war die Umsetzung beendet, da kein Peroxid mehr iodometrisch nachweisbar war.

Nach der gaschromatographischen Analyse betrug die Ausbeute an Cyclohexanon 98% und die an 1,2-Octenoxid 71%, jeweils bezogen auf das eingesetzte Cyclohexanonperoxid.

*Beispiel 3*

Herstellung von Propylenoxid (Methyloxiran)

Ein Gemisch aus 10 g (238 mmol) Propylen, 59,6 g (= 23,8 mmol Peroxid) der Cyclohexanonperoxid-Lösung gemäss Beispiel C und 6 g des Katalysators gemäss Beispiel B wurde 30 min lang im Schüttelautoklaven auf 80°C erhitzt.

Das Reaktionsgemisch enthielt kein Peroxid mehr, und die Ausbeuten an Propylenoxid und Cyclohexanon betrugen nach der GC-Analyse 63 bzw. 96%, jeweils bezogen auf das eingesetzte Peroxid.

*Beispiel 4*

Herstellung von Cyclohexenoxid (1,2-Tetramethylenoxiran)

Eine Suspension aus 1,5 g des Katalysators gemäss Beispiel B und 82 g (1 mol) Cyclohexen wurden bei 80°C unter Rühren innerhalb von 30 min mit 53 g (= 21 mmol Peroxid) der Cyclohexanonperoxidlösung gemäss Beispiel C versetzt. Nach weiteren 15 min betrug der Peroxidumsatz 90% und die hierauf bezogenen Ausbeuten an Cyclohexenoxid und Cyclohexanon nach GC-Analyse 94 bzw. 98%.

**Patentansprüche**

1. Verfahren zur Herstellung von Oxiranen durch Umsetzung der entsprechenden olefinisch ungesättigten Verbindungen mit organischen Peroxiden in flüssiger Phase sowie in Gegenwart von Trägerkatalysatoren als Epoxidierungskatalysatoren, die in der aktiven Masse vornehmlich Titandioxid enthalten und deren Trägermaterial vornehmlich aus Siliciumdioxid oder Silikaten besteht, dadurch gekennzeichnet, dass man hierzu Cyclohexanonperoxid als organisches Peroxid verwendet.

2. Verfahren nach Anspruch 1, dadurch gekennzeichnet, dass man solche Trägerkatalysatoren verwendet, die zur Aktivierung mit organischen Silicium-Verbindungen nachbehandelt wurden.

**Claims**

1. A process for the preparation of an oxirane by reacting the corresponding olefinically unsaturated compound with an organic peroxide in the liquid phase and in the presence of a supported catalyst, as epoxidation catalyst, whose active material chiefly contains titanium dioxide and whose carrier mainly consists of silicon dioxide or a silicate, wherein the organic peroxide used is cyclohexanone peroxide.

2. A process as claimed in claim 1, wherein the supported catalyst used is one which has been activated by aftertreating it with an organic silicon compound.

**Revendications**

1. Procédé de préparation d'oxirannes par réaction de composés à insaturation oléfinique appropriés en phase liquide avec des peroxydes organiques, en présence en tant que catalyseurs d'époxydation de catalyseurs sur support, dont la matière active est principalement constituée de dioxyde de titane et dont les matériaux supports sont de préférence du dioxyde de silicium ou des silicates, caractérisé en ce que le peroxyde organique est le peroxyde de cyclohexanone.

2. Procédé suivant la revendication 1, caractérisé en ce que l'on met en œuvre des catalyseurs sur support activés par un post-traitement avec des dérivés organiques du silicium.